# FASCICULE DE BREVET EUROPEEN

(11) **EP 0 488 916 B1**
(45) Date de publication et mention de la délivrance du brevet: **15.01.1997**
(21) Numéro de dépôt: 91420422.7
(22) Date de dépôt: 28.11.1991
(51) Int. Cl.: C12P 7/44, C12N 15/55, C12P 41/00

(54) **Procédé de synthèse enzymatique d'adipate d'ammonium**
Enzymatisches Herstellungsverfahren für Ammoniumadipat
Process for enzymatic preparation of ammonium adipate

(30) Priorité: 28.11.1990 FR 9014853
(43) Date de publication de la demande: 03.06.1992
(73) Titulaire: RHONE-POULENC FIBER AND RESIN INTERMEDIATES, 92408 Courbevoie (FR)
(72) Inventeur: Yeh, Patrice, F-75005 Paris (FR); Mayaux, Jean-François, F-92260 Fontenay aux Roses (FR); Cerbelaud, Edith, F-69350 La Mulatière (FR); Petre, Dominique, F-69009 Lyon (FR)
(74) Mandataire: Ropital-Bonvarlet, Claude

(56) Documents cités:
- EP-A- 330 529
- MICROBIOLOGIE ALIMENTS NUTRITION, vol. 4, 1986, Chatenay-Malabry (FR); M. MAESTRACCI et al., pp. 19-24
- JOURNAL OF BACTERIOLOGY, vol. 172, no. 12, 1990, Baltimore, MD (US); J.-F. MAYAUX, pp. 6764-6773

## Description

La présente invention concerne un procédé de synthèse enzymatique d'adipate d'ammonium.

Plus précisément, l'invention concerne un procédé de synthèse d'adipate d'ammonium par hydrolyse d'adipamide et/ou d'adipamate d'ammonium au moyen d'un catalyseur enzymatique spécifique.

On sait que l'adipate d'ammonium est un produit particulièrement intéressant car il peut être transformé en acide adipique, un produit lui-même largement utilisé pour la préparation du Nylon.

La synthèse de l'adipate d'ammonium par hydrolyse enzymatique de l'adipamide est déja connue dans son principe.

Ainsi, parmi les microorganismes chez lesquels on a pu démontrer l'éxistence de cette activité enzymatique, on peut plus particulièrement citer les souches appartenant au genre Brévibactérium, et notamment Brévibactérium R 312 (voir à ce sujet ARNAUD et al, "Etude de l'activité amidasique de quelques bactéries", in Folia Microbiologica, 1976, 21, page 178-185). En outre, l'éxistence au sein de Brévibactérium d'une amidase dite "à activité générale" directement utilisable pour la bioconversion des amides en sels d'acides, et notamment de l'adipamide en adipate d'ammonium, a également déja été mise en évidence (voir à ce sujet MAESTRACCI et al, in Microbiologie Aliments-Nutrition, 1986, Vol 4, page 19-24).

Toutefois, il s'avère que l'activité enzymatique de Brévibactérium R 312, ou de son amidase à activité générale décrite ci-dessus, est insuffisante pour permettre une production élevée, et donc rentable, en adipate d'ammonium à partir de l'adipamide.

L'un des buts poursuivis par la présente invention réside donc dans la mise à disposition d'un procédé de synthèse enzymatique d'adipate d'ammonium présentant des rendements améliorés.

Or, il a été trouvé par la demanderesse que ce but pouvait être atteint si l'on faisait appel à des enzymes convenablement sélectionnées, utilisées soit telles quelles soit, de préférence, sous la forme de microorganismes recombinés les générant.

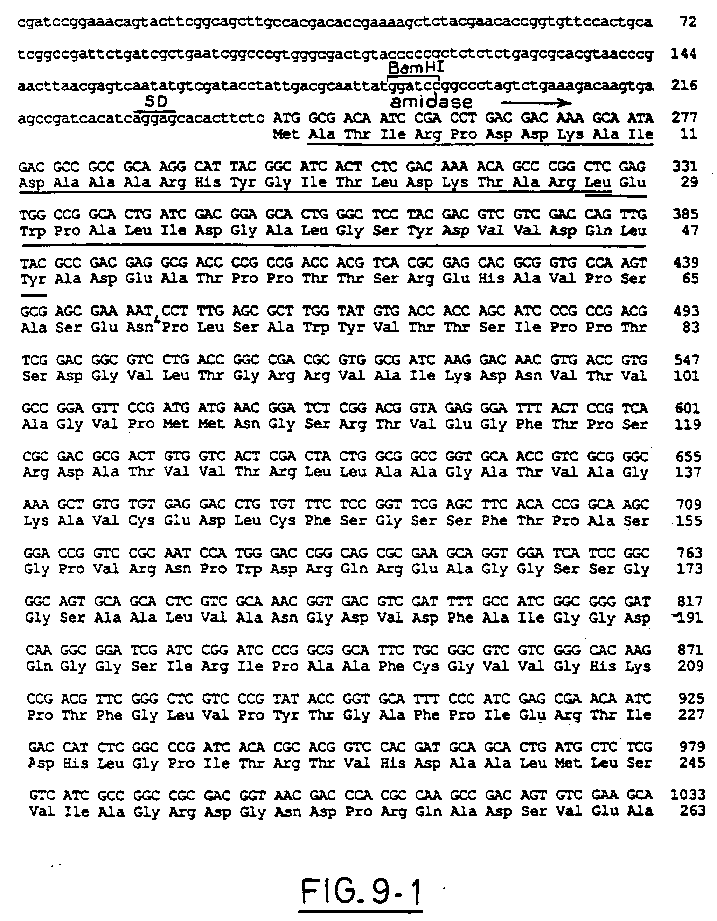

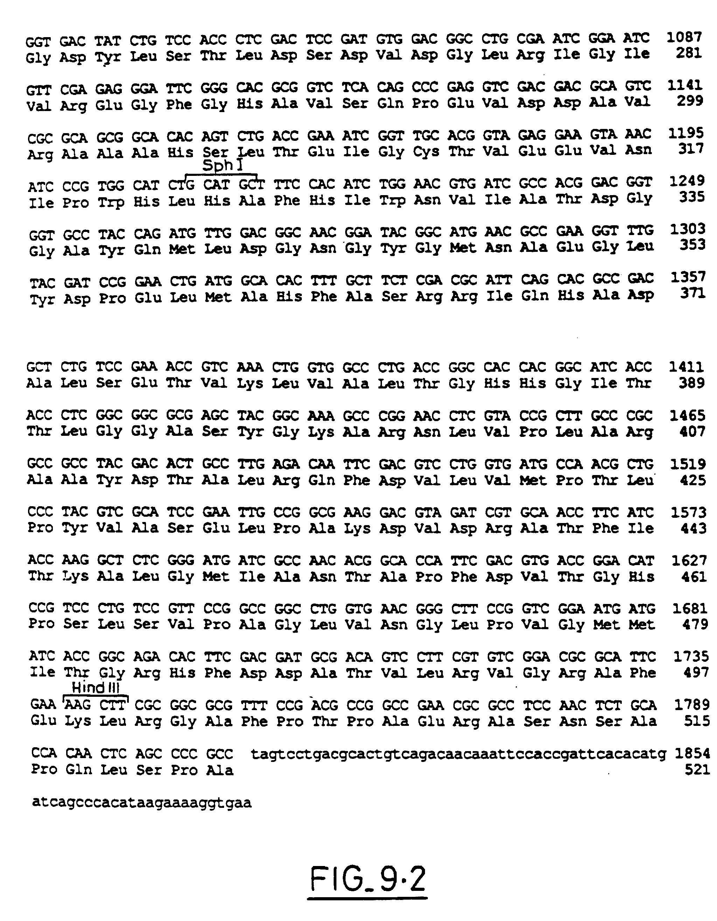

Plus précisément, il est maintenant proposé un nouveau procédé de synthèse d'adipate d'ammonium par hydrolyse de l'adipamide et/ou de l'adipamate d'ammonium au moyen d'un polypeptide ayant une activité amidase, ou d'un microorganisme recombiné générant ledit polypeptide, ledit procédé étant caractérisé par le fait que l'on utilise :
(i) un polypeptide codé par une séquence d'ADN choisie parmi :
   - la séquence codant pour l'amidase de Brévibactérium R 312 représentée à la figure 9.
   - un analogue de cette séquence résultant de la dégénérescence du code génétique
   - un ADN hybridant avec l'une de ces séquences ou avec un fragment de celles-ci et codant pour un polypeptide ayant une activité amidase.
ou (ii) un microorganisme recombiné générant ledit polypeptide.

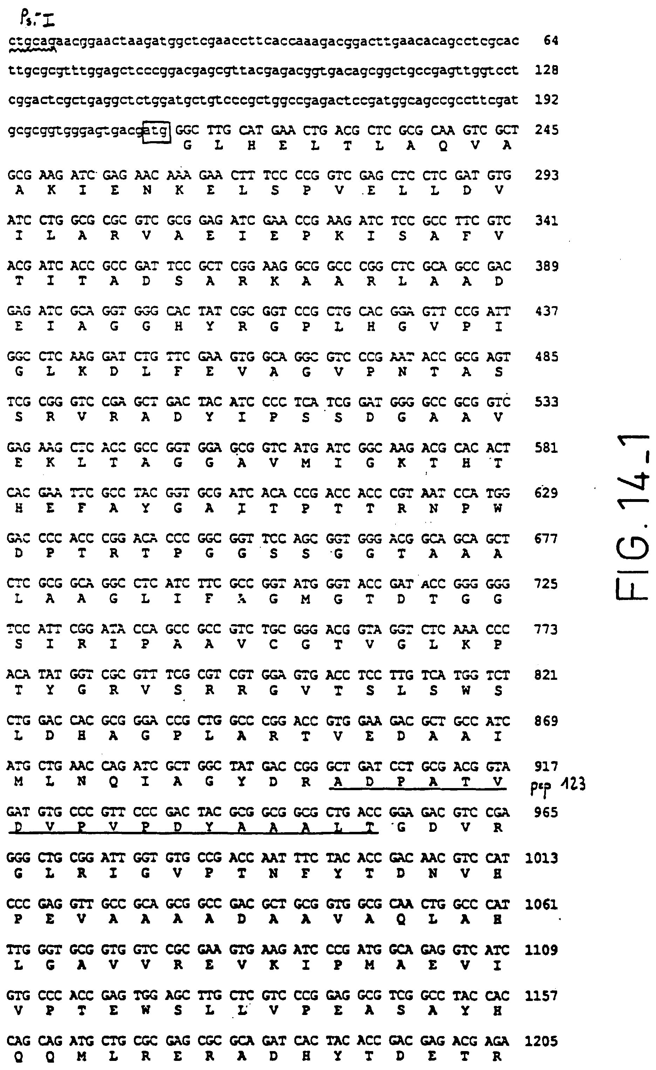

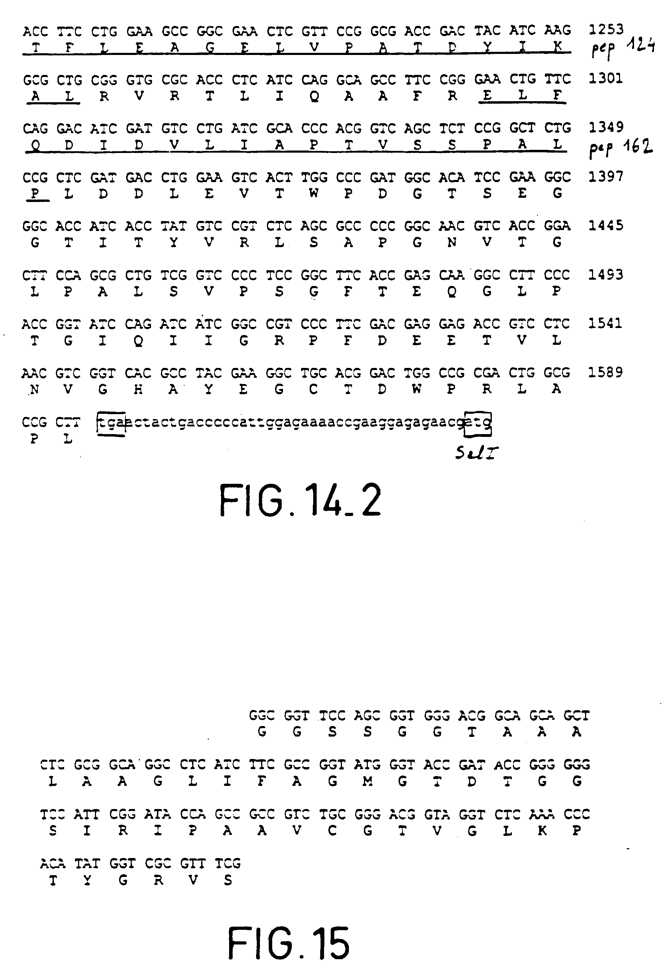

Selon un mode particulier de réalisation de l'invention, on utilise un polypeptide (ou un microorganisme recombiné générant ce polypeptide) codé par la séquence telle que représentée à la figure 14, ou par un variant de cette séquence. On entend ici par variant toute séquence qui en dépit de quelques altérations résultant par exemple de mutations, délétions ou insertions, ou bien encore de la dégénérescence du code génétique, préserve les propriétés de la séquence initiale.

Comme indiqué ci-après, de telles séquences peuvent être trouvées dans des souches du type Rhodococcus.

Selon la présente invention, la séquence d'ADN représentée à la figure 14 est considérée comme hybridant avec la séquence d'ADN représentée à la figure 9.

Les polypeptides tels que définis ci-dessus, ainsi que les microorganismes recombinés générant ces polypeptides, qui sont utilisés dans le procédé selon la présente invention, ont déja été décrits dans la demande de brevet française déposée sous le numéro 8916332 au nom de RHONE-POULENC SANTE. Ils ne font donc pas l'objet, en tant que tels, de la présente invention.

D'une manière très générale, cette demande de brevet non encore publiée concerne des polypeptides possédant une activité amidase dite éniantiosélective, les séquences d'ADN permettant leur expression, un procédé pour leur préparation, ainsi que leur utilisation comme catalyseurs pour hydrolyser énantiosélectivement des racémiques d'amides en un acide soit de forme S, soit de forme R. Ces polypeptides, ainsi que le matériel génétique permettant leur expression, ont été globalement obtenus conformément aux exemples qui seront donnés ci-après.

On notera donc que les procédés d'obtention de tels polypeptides, ou du matériel génétique permettant leur expression, ou des microorganismes recombinés les générant, ne constituent pas l'objet de la présente invention.

La présente invention est en fait basée sur la découverte que de tels polypeptides à activité amidase énantiosélective présentent également la propriété remarquable de pouvoir hydrolyser l'adipamide et/ou l'adipamate d'ammonium en adipate d'ammonium avec des rendements particulièrement élevés. La présente invention a donc pour unique objet une utilisation nouvelle de polypeptides particuliers.

Les séquences d'ADN qui codent pour les polypeptides utilisés dans le procédé selon l'invention peuvent être obtenues de diverses façons. La stratégie générale consiste à cloner, à l'aide de sondes nucléotidiques élaborées à partir du polypeptide purifié, le fragment d'ADN génomique codant pour le polypeptide recherché. A l'aide de différentes méthodes, telles que l'élongation d'amorces, la restriction, l'insertion d'adaptateurs ou la ligature avec des oligonucléotides de raccord, on construit un insert nucléotidique contenant la séquence d'ADN désirée. Celle-ci peut alors être cartographiee et séquencée par les techniques connues.

D'autres techniques peuvent être également envisagées, comme l'utilisation d'ADN et/ou de la synthèse chimique partielle ou totale.

Ces techniques sont connues et les structures décrites aux figures 9 et 14 permettent à l'homme de métier d'isoler une séquence équivalente, dans tout microorganisme, avec les moyens connus en la matière.

Les polypeptides, dont la structure se déduit des séquences d'ADN ci-dessus, et qui possèdent une activité amidase, peuvent être obtenus à partir de différents microorganismes, et notamment de Brévibactéries ou de bactéries du genre Rhodococcus. Plus précisément encore, ces polypeptides sont préparés par extraction et purification à partir de cultures de microorganismes naturels ou recombinants, la purification étant réalisée par une succession d'étapes consistant à préparer un extrait enzymatique brut à partir de la culture cellulaire, à fractionner cet extrait au sulfate d'ammonium, et à le purifier par différentes chromatographies et filtrations sur gel. Les détails de ces étapes sont donnés dans les exemples.

Les polypeptides purifiés peuvent ensuite être séquencés, leurs gênes clônés et mis à exprimer dans différents microorganismes recombinés (microorganismes hôtes), selon des techniques courantes de biologie moléculaire (technique des ADN recombinants). Plus précisément, les microorganismes transformés contiennent au moins une cassette d'expression des séquences d'ADN telles que représentées aux figures 9 et 14, ces cassettes étant de préférence constituées par l'une desdites séquences d'ADN sous la dépendance de séquences d'ADN assurant son expression dans l'hôte considéré. Il peut s'agir d'une cassette intégrée directement dans le génome de l'hôte, ou d'une cassette insérée dans un plasmide qui comporte, en outre, une origine de replication plasmidique active dans l'hôte et un moyen de sélection.

Les séquences d'ADN assurant l'expression des séquences d'ADN susmentionnées comportent de préférence une région d'initiation de la transcription et de la traduction.

Selon ce procédé, les régions de démarrage de la transcription et de la traduction contiennent un promoteur et un site de fixation des ribosomes. Ces régions peuvent être homologues ou hétérologues du polypeptide produit.

Le choix de ces régions dépend notamment de l'hôte utilisé. En particulier, lorsqu'il s'agit de microorganismes hôtes procaryotes, le promoteur hétérologue peut être choisi parmi les promoteurs bactériens forts, tels que le promoteur de l'opéron tryptophane Ptrp, le promoteur de l'opéron lactose Plac, le promoteur droit du phage lambda P_{R}, le promoteur gauche du phage lambda P_{L}, les promoteurs forts de phages de corynébactéries, ou encore les promoteurs homologues de corynébactéries. Plus particulièrement, dans le cas du promoteur droit du phage lambda, la forme thermosensible pourra être préférée P_{R}CIts. Dans le cas de microorganismes eucaryotes, telles les levures, les promoteurs peuvent être issus de gènes glycolytiques de levure, tels les gènes codant pour la phosphoglycérate kinase (PGK), la glycéraldéhyde-3-phosphate déshydrogénase (GPD), la lactase (LAC4), l'énolase (ENO).

Concernant les sites de fixation des ribosomes, celui dérivé du gène CII de lambda ainsi que ceux dérivés de gènes homologues de corynébactéries sont utilisés préférentiellement lorsque le microorganisme hôte est procaryote.

Une région permettant une terminaison de la traduction et de la transcription fonctionnelle dans l'hôte envisagé peut être positionnée en 3' de la séquence codante. Le plasmide comprend également un'ou plusieurs marqueurs permettant de sélectionner l'hôte recombinant. Les marqueurs préférés sont des marqueurs dominants, c'est-à-dire conférant une résistance à des antibiotiques comme l'ampicilline ou la streptomycine ou à d'autres produits toxiques.

Parmi les microorganismes hôtes utilisés, on peut citer notamment les entérobactéries telles que E.Coli, les bactéries corynéformes telles que celles appartenant aux genres Corynebactérium, Brévibacterium, ou Rhodococcus.

Bien entendu, sur le même principe on peut utiliser d'autres types cellulaires.

L'adipate d'ammonium est, selon l'invention, préparé simplement en mettant l'adipamide et/ou l'adipamate d'ammonium (réactifs) en présence d'un polypeptide ou d'un microorganisme recombiné tels que décrits précédemment. On opère généralement à température ambiante.

Selon un mode particulier de réalisation de l'invention, le polypeptide ou le microorganisme recombinant contenant le polypeptide, sont immobilisés sur ou dans un support solide.

Les exemples qui suivent permettent d'illustrer les caractéristiques et les avantages de la présente invention, sans toutefois en limiter la portée.

### DESCRIPTION DES FIGURES

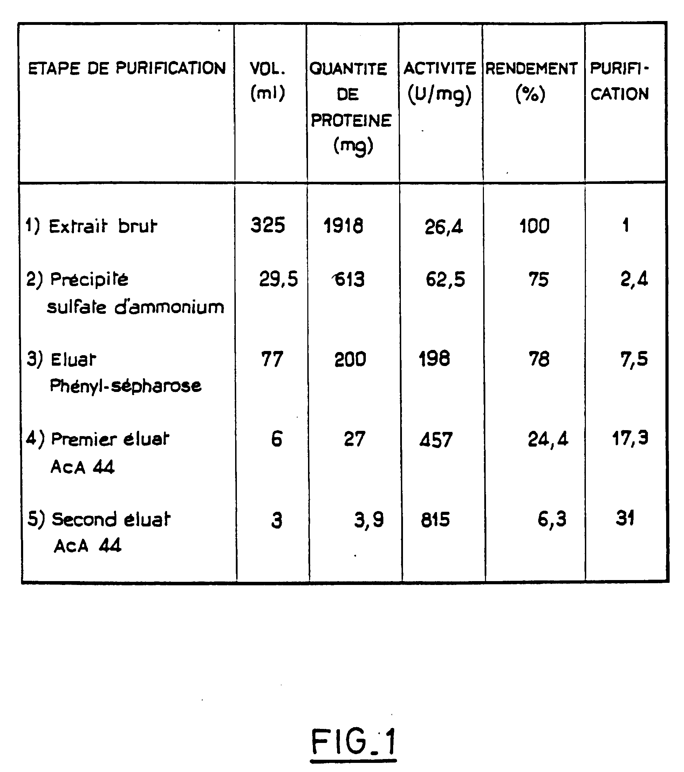

- Figure 1 : Table décrivant les différentes étapes de la purification de l'amidase énantiosélective de Brevibactérium R 312.
   a - à partir de 40 g de cellule humide, après précipitation au sulfate de streptomycine ;
   b - une unité (U) est égale à 1 µmole d'acide HPPA formé par heure dans les conditions décrites ci-après.
- Figure 2 : a - Séquences peptidiques (N-terminale et interne) obtenues à partir de la solution purifiée de Brevibacterium R 312
   b - Sonde oligonucléotidique réalisée à partir du fragment interne.
- Figure 3 : a - Stratégie d'élaboration de la sonde sq 918, à partir du fragment N-Terminal
   b - Sonde sq 918 obtenue.
- **Figure 4** : a - Profil d'hybridation de la sonde sq 918 avec l'ADN génomique total de Brevibacterium R 312 digéré par les enzymes ECORI, HindIII, kpnI, PstI et SphI
   b - Profil d'hybridation de la sonde sq 762 avec l'ADN génomique total de Brevibacterium R 312 digéré avec les mêmes enzymes que (a).
- **Figure 5** : Cartes de restriction des plasmides pXL1650 et pXL1651.
- **Figure 6** : Carte de restriction du fragment PstI de 5,4 kb contenant le gène de l'amidase énantiosélective de Brevibacterium R 312.
- **Figure 7** : Stratégie de séquençage du fragment BamHI-PstI contenant le gène de l'amidase énantiosélective de Brevibacterium R 312.
- **Figure 8** : Analyse des phases ouvertes du fragment séquencé.
- **Figure 9** : Séquences nucléotidique et peptidique de l'amidase énantiosélective de Brevibacterium R 312.
- **Figure 10** : Carte de restriction du plasmide pXL1724.
- **Figure 11** : Carte de restriction du plasmide pXL1751.
- **Figure 12** : Carte de restriction du plasmide pXL1752.
- **Figure 13** : Gel de polyacrylamide -SDS à 12,5 % après coloration au bleu de Coomassie démontrant l'expression de l'amidase énantiosélective de Brevibacterium R 312 à partir des souches coli et E103S. Chaque piste correspond à une quantité de protéine équivalente à 60 µl de la culture à une densité optique à 610 nm de 2,1 (E103S) et 0,7 (coli B). T), fraction soniquée ; S), fraction soluble ; C), fraction insoluble. Les plasmides témoins (pXL1029 et pXL906) contiennent respectivement le gène de l'lL1béta sous le contrôle du promoteur PRCits et Ptrp.
- **Figure 14** : Séquences nucléotidique et peptidique de l'amidase énantiosélective d'un Rhodococcus.
- **Figure 15** : Séquences nucléotidique et peptidique de la région 150-200.
- **Figure 16** : Etudes d'homologies de séquences : recherche du site actif.

Légende : Homologie entre les amidases énantiosélectives de B. R312 (ligne du haut) et celle représentée à la figure 14 (ligne du bas). On remarque une homologie stricte de 50 % entre les résidus 150 et 300 de l'amidase de R312, allant jusqu'à ≃ 67 % entre les résiduts 158 et 215 (zone précédemment définie comme étant le site actif probable sur la base d'homologies entre l'amidase de R312 et d'autres amidases).

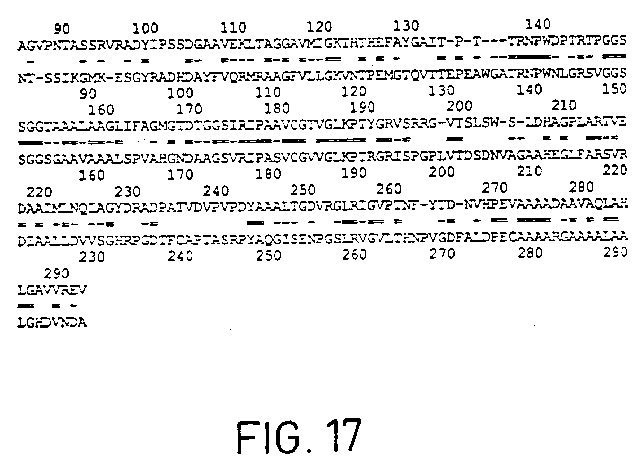

- **Figure 17** : Etudes d'homologies de séquences : recherche du site actif.

Légende : Homologie entre la séquence présentée figure 14 Oigne du haut) et la 6- aminohexanoate cyclic dimer hydrolase de Flavobacterium sp. K172 (ligne du bas). L'homologie est essentiellement située au niveau du site actif présumé de ces amidases (résidus 136-194).

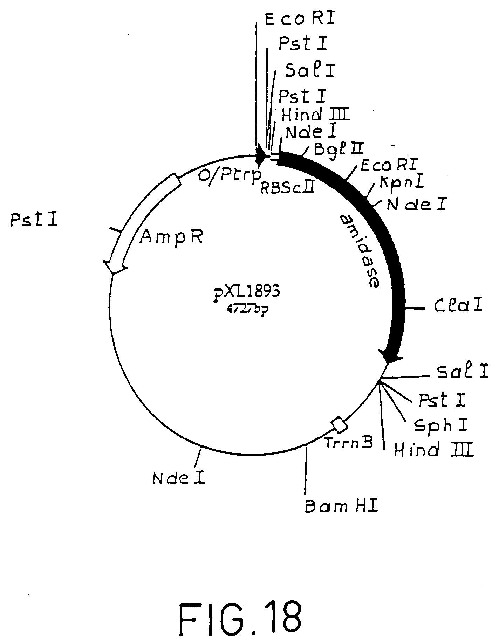

- **Figure 18** : Vecteurs d'expression dans E. coli des séquences Amd présentées aux figures 14 et 15.

Légende : Vecteur d'expression E. coli de l'amidase R312 sous le contrôle du promoteur tryptophane et du RBS cII. Le même vecteur, pX1894, a été obtenu où le gène est placé sous le contrôle du promoteur PR et du répresseur CI^{ts} (pXL 1394).

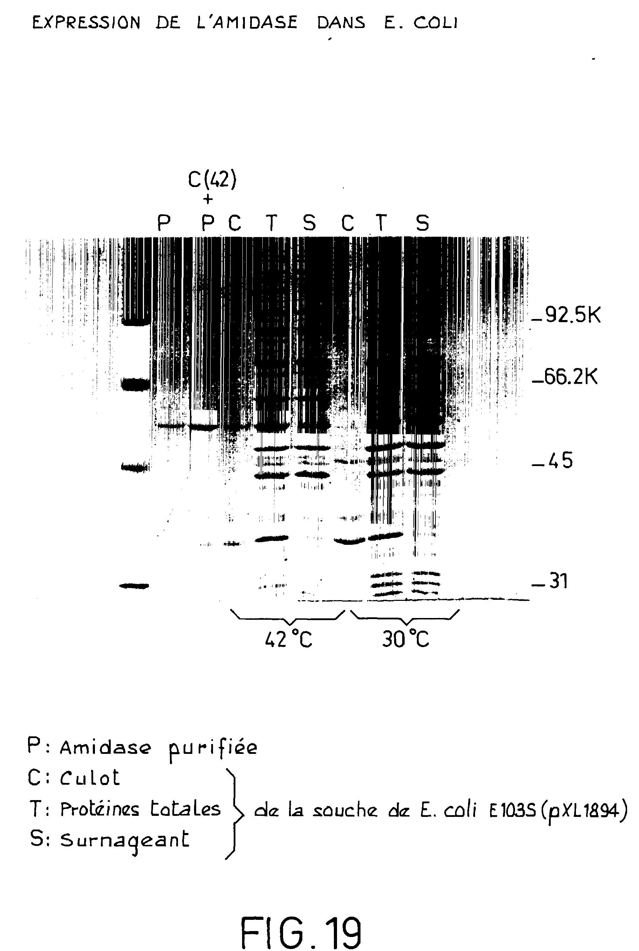

- **Figure 19** : Résultat d'expression avec la souche E. coli E103S transformée par le plasmide pXL 1894.

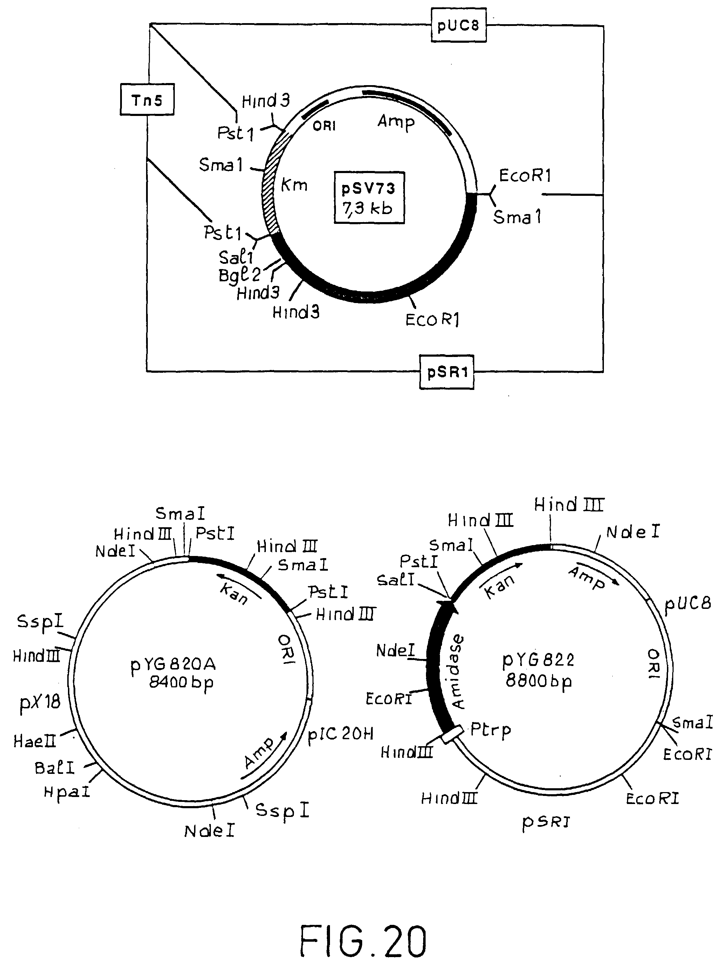

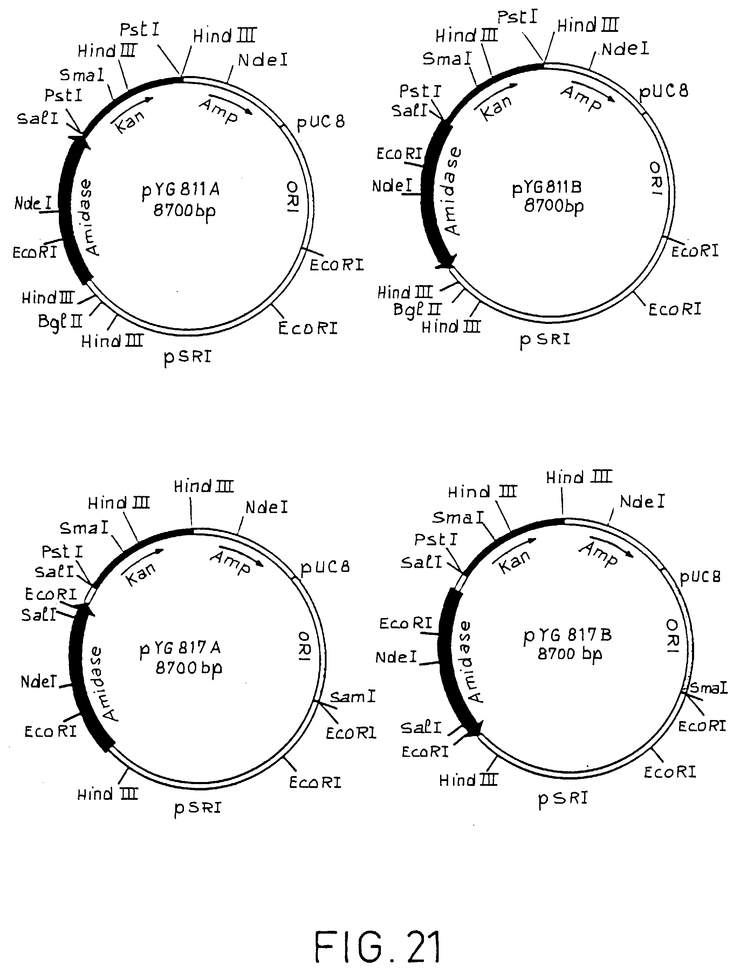

- **Figures 20 et 21 :** Vecteurs d'expression dans les corynébactéries des séquences Amd présentées aux figures 14 et 15.

### PLASMIDES DE DEPART

Le plasmide pXL534 dérive de pXL276 (qui contient Ptrp-RBSc11ΔtR1-HSA: brevet européen n° 86 400617.6) par délétion à l'aide de l'enzyme BA131 à partir du site EcoRV, d'un fragment de 2,1 kb, et ligature sur linker BamHI.

Le plasmide pXL820 dérive du plasmide pXL534 par excision du fragment EcoRI-NdeI portant le Ptrp et le RBScIIΔtRI, et insertion d'un fragment EcoRI-NdeI contenant le promoteur PRCIts et le site RBS cIIΔ tRI. Ce dernier est issu du plasmide pRK248 cIts (Bernard et al, Gene 5, 59-76) grâce aux étapes suivantes :
. Excision d'un fragment BgII-BgII de pRK248clts contenant PRclts.
. Insertion de ce fragment au site BamHI du plasmide pUC19.
. Linéarisation de pUCI9 ainsi obtenu, par CIaI, et les extrémités sont remplies avec l'ADN polymérase de Klenow.
. Digestion supplémentaire par SmaI, et ligature.
. De cette nouvelle construction est excisé un fragment EcoRI-SalI contenant PRcIts, qui est inséré dans pXL534 ouvert par ces deux enzymes.
. Le fragment EcoRI-NdeI est ensuite excisé de ce plasmide et il contient PRcIts-RBS cII Δ tRI.

### EXEMPLE 1 - Identification et purification de l'amidase énantiosélective de Brevibacterium R312.

### 1.1. - Identification

Le R,S-(hydroxy-4 phénoxy)-2 propionamide, dérivé aryloxy-2 propionamide, est meilleur substrat de l'amidase énantiosélective que les dérivés aryl-2 propionamides, notamment le phényl-2 propionamide et le (benzoyl-3 phényl)-2 propionamide. De plus, la sélectivité de l'amidase vis-à-vis de l'énantiomère R de l'HPPAmide est représentative de la sélectivité vis-à-vis de l'énantiomère S des dérivés aryl-2 propionamide.

En conséquence, l'activité enzymatique énantiosélective a été détectée en utilisant comme substrat l'(hydroxy-4 phénoxy)-2 propionamide (HPPAmide). La réaction est conduite à 25°C sous agitation dans un tampon phosphate sodium 50 mM, pH 7,0, en présence de Brevibacterium R312 et est arrêtée par addition d'un mélange acide phosphorique 0,05M, acétonitrile et HCl IN 55/40/5 (v/v). La culture est ensuite centrifugée et le surnageant est analysé en CLHP en phase inverse (Hibar-Merck RP-18 ; 5 µm). L'élution est effectuée avec une solution 85/15 (v/v) d'acide phosphorique 0,005M et d'acétonitrile et les concentrations respectives de HPPAmide et HPPAcide sont mesurées grâce à la position des pics d'élution et comparativement à un standard. L'excès énantiomérique, défini comme le rapport (R-S)/(R+S) x 100 dans lequel R et S sont respectivement des concentrations en énantiomère R et S de l'HPPAcide, se déduit soit de mesures polarimétriques (utilisant l'absorption à 589nm du sodium) soit de l'analyse CLHP sur colonne chirale.

Les activités obtenues avec les cellules entières et un extrait soluble sont respectivement de 15U/mg et 24U/mg de protéine. (1U = 1µmole d'HPPAcide formée par heure). L'excès énantiomérique de l'HPPAcide R formé est de 95%. Ces résultats montrent que Brevibacterium R312 possède une activité amidase énantiosélective capable d'hydrolyser les aryl-2 propionamides racémiques en acides S correspondants. Cela a été vérifié avec les hydrolyses du phényl-2 propionamide racémique, et du (benzoyl-3 phényl)-2 propionamide racémique qui donnent respectivement les acides S correspondants avec des excès énantiomériques supérieurs 93%.

### 1.2. - Purification

La purification est réalisée à 4°C.

Les cellules (40g poids sec de Brevibacterium R312) sont décongelées et reprises dans 300 ml de tampon A (Phosphate de sodium 50mM, pH 7, β -mercaptoéthanol 5 mM). Les cellules sont ensuite cassées par ultrasons et les débris membranaires sont éliminés par centrifugation à 20000 g pendant 30 minutes. 25 ml d'une solution de sulfate de streptomycine 10% sont ajoutés lentement sous agitation à 30 ml de surnageant. Après 45 minutes, la solution est clarifiée comme ci-dessus et le surnageant est traité au sulfate d'ammonium. La fraction protéique précipitant entre 30,8% et 56,6% de saturation au sulfate d'ammonium est récupérée par centrifugation et dissoute dans 35 ml du tampon A, avant d'être dialysée plus longuement contre ce même tampon. La solution ainsi obtenue est alors ajustée à 20% de la saturation en sulfate d'ammonium, de nouveau centrifugée et appliquée sur une colonne phényl-Sépharose CL-4B (Pharmacia) équilibrée avec du tampon A à 20% de la saturation en sulfate d'ammonium. Les fractions protéiques contenant l'activité enzymatique sont ensuite éluées avec le même tampon, puis concentrées par ultrafiltration avec une cellule AMICON DIAFLO PM 10 jusqu'à un volume de 18 ml. Du glycérol 10% est ensuite ajouté à la fraction concentrée et la solution obtenue est chargée sur une colonne Ultrogel AcA 44 (IBF-biotechnics France) préalablement équilibrée en Tris-HCl 50 mM, pH 7,0, NaCI 100 mM. Les fractions protéiques contenant la plus grande activité spécifique (environ 32% de l'activité totale chargée sur la colonne) sont collectées, concentrées et soumises à une étape de filtration supplémentaire sur le même gel. De la même façon, les fractions présentant la plus grande activité spécifique (environ 30% des protéines appliquées sur la colonne) ont été analysées en SDS-PAGE et stockées. L'énantiosélectivité de la protéine ainsi purifiée a également été déterminée.

Ces étapes de purification ont permis d'obtenir une enzyme pure à plus de 80% avec une activité spécifique de 815 U/mg. A ce stade, une bande majoritaire d'un poids moléculaire apparent de 59 ± 5 KD, correspondant à au moins 80% des protéines totales, est visible en SDS-PAGE. De plus, l'activité amidase éluée de l'Ultrogel AcA 44 correspond également à un poids moléculaire de 63 ± 5 KD, indiquant que l'enzyme se présente apparemment sous forme monomérique.

### Exemple 2 - Clonage de l'amidase énantiosélective de Brevibacterium R312

### 2.1. - Obtention de séquences protéiques

Les séquences peptidiques correspondant respectivement à l'extrêmité N-terminale (27 résidus) et à un fragment trypsique interne (21 résidus) de l'amidase énantiosélective de Brevibacterium R312 ont été déterminées à partir de l'enzyme ainsi purifiée.

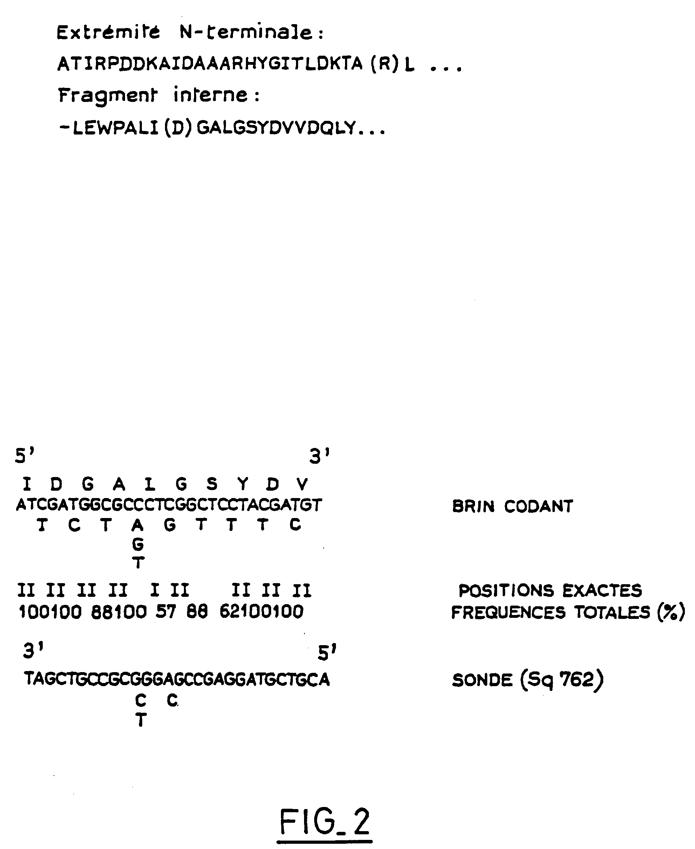

Pour cela, 3 nmol. de la préparation d'amidase ont été réduites et carboxyméthylées. Le composé protéique majoritaire est ensuite déssalé, et purifié jusqu'à homogénéité par chromatographie CLHP en phase inverse. La séquence N-terminale est ensuite déterminée selon la méthode de dégradation séquentielle automatique d'Edman, en utilisant un appareil "Applied Biosystems Model 470A". De cette manière, la séquence présentée figure 2a est obtenue. Afin d'obtenir une séquence supplémentaire interne, la même quantité de protéine est soumise à une digestion trypsique. Les fragments réduits et carboxyméthylés sont ensuite séparés par chromatographie CLHP en phase inverse (2,1x10mm ; débit ; 0,2ml/min), en utilisant le tampon d'élution suivant gradient de 0 à 50% d'acétonitrile dans 0,07% d'acide trifluoroacétique. Un peptide élué dans un pic bien séparé (à 40,8% d'acétonitrile) est séquence (Figure 2a).

### 2.2. Elaboration des sondes nucléotidiques

Deux types de stratégies ont été poursuivies pour la construction des sondes nucléotidiques.

Dans la première stratégie, une sonde 29-mer (59% d'homologie minimale) a été construite, tenant compte de l'usage des codons dans l'opéron tryptophane de Brevibacterium lactofermentum (séquence de 7,7 kb contenant 6 cistrons : Matsui et al Mol. Gen. Genet. 209 p. 299, 1987) et d'après la séquence IDGALGSYDV du fragment interne (présentant une dégénérescence moyenne moins importante). Le brin non-codant a été synthétisé en considérant la relative neutralité thermodynamique d'appartements G = T et en introduisant quelques dégénérescences pour maximiser la fréquence théorique moyenne des codons considérés (88% par rapport à l'usage des codons choisis). Les considérations conduisent à porter le GC % de la sonde à environ 69%. La sonde obtenues (sq 762) est donnée à la figure 2b.

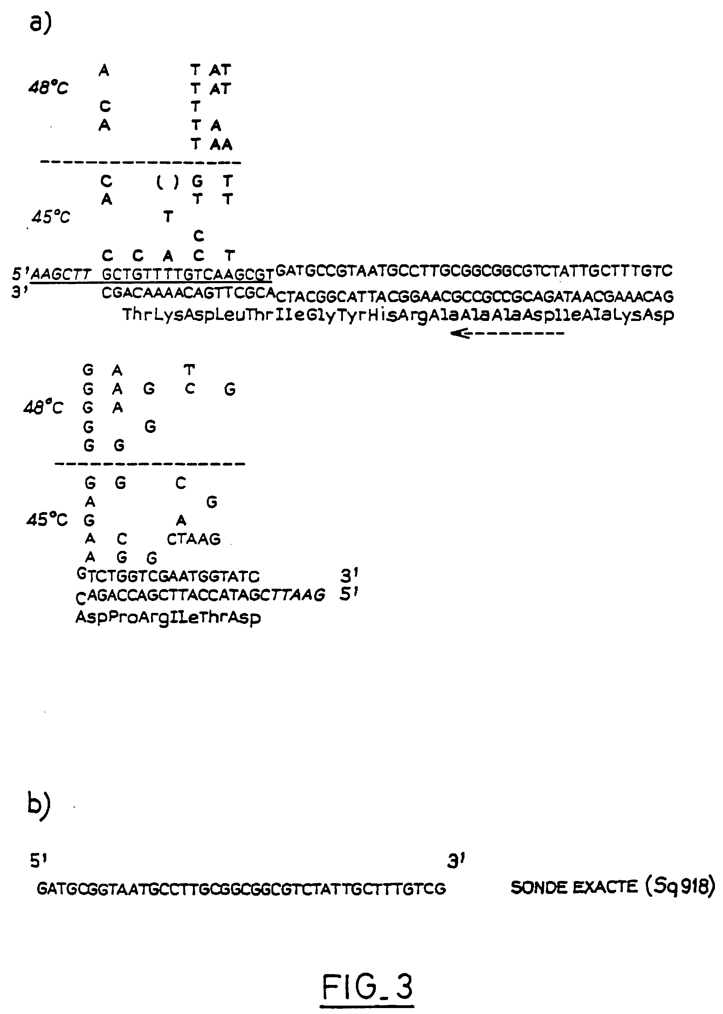

Dans un deuxième type de stratégie, on a mis en oeuvre la méthode PCR décrite par Girgès et al (Nucleic Acids Res. 16, p.10371, 1988) pour obtenir une sonde nucléotidique exacte à partir d'un peptide correspondant à des codons hautement dégénérés. Pour cela, des oligonucléotides synthétiques 25-mer (voir séquence soulignée sur la figure 3) correspondant à toutes les possibilités de clonage des cinq premiers ou cinq derniers codons de la séquence peptidique N-terminale et comportant des sites de restriction à leurs extrêmités 5′ (respectivement EcoRI et HindIII) ont été utilisés pour amorcer une amplification enzymatique de l'ADN génomique de Brevibacterium R312. Après 30 cycles d'amplification le fragment candidat est purifié sur gel puis inséré entre les sites HindIII et EcoRI du bactériophage M13mp19. Un certain nombre de clones, obtenus après clonage du fragment obtenu à deux températures différentes d'hybridation des amorces (45°C et 48°C), ont ensuite été séquencés et comparés. Les résultats sont indiqués sur la figure 3. Cette figure montre que, à part les dégénérescences introduites par les amorces, on a bien amplifié un fragment d'ADN (unique entre les amorces) codant pour l'extrêmité N-terminale de l'amidase. Un oligonucléotide synthétique 40-mer correspondant à ce fragment interne a donc été utilisé pour la suite du clonage, comme sonde exacte de l'extrêmité N-terminale de l'amidase. La séquence de ce fragment sq 918 est indiquée figure 3.

Les deux sondes ainsi obtenues ont été marquées au ³²p selon la méthode de phosphorylation en 5′.

### 2.3. Clonage du gène de l'amidase énantiosélective de Brevibacterium R312

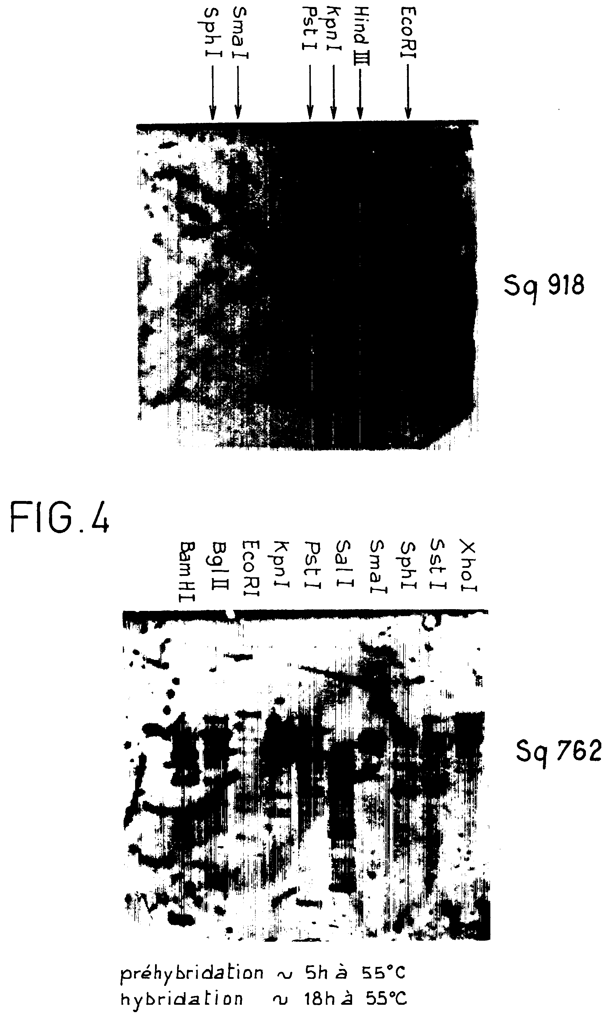

La stratégie suivie a consisté tout d'abord à vérifier la spécificité des sondes synthétisées et à déterminer la nature du fragment d'ADN génomique à cloner par Southern Blot. Brièvement, de l'ADN génomique de Brevibacterium R312 a été digéré alternativement par plusieurs enzymes de restriction correspondant à des sites utilisables pour le clonage, en particulier, à des sites présents dans le multi-site de clonage des plasmides de la série pUC. Notamment, l'enzyme PstI a été utilisé. Après électrophorèse sur gel d'agarose et transfert sur membrane de nylon, les diverses digestions ont été hybridées aux sondes sq 762 et sq 918. Les résultats présentés figure 4 montrent que les deux sondes sont caractérisées par une spécificité suffisante dans des conditions d'hybridation (au plus un fragment hybridant pour chaque digestion). De plus, dans la mesure où les deux sondes permettent d'obtenir à peu près le même profil d'hybridation, on peut penser que les signaux d'hybridation sont bien spécifiques du gène recherché, et que le peptide interne obtenu après hydrolyse trypsique est très proche de l'extrêmité N-terminale. Les empreintes d'hybridation montrent en particulier l'existence d'un fragment unique PstI de 5,4 kpb environ qui hybride très fortement avec les deux sondes. Il a alors été décidé de cloner ce fragment. Pour cela, tous les fragments de taille comprise entre 4,6 et 5 kpb et 6 à 6,5 kpb environ, résultant d'une digestion génomique totale de Brevibacterium R312 par PstI ont été purifiés sur agarose, électroélués, puis ligaturés au vecteur p UC 19, préalablement digéré par PstI. Après transformation dans la souche d'DH5α d'E.coli, 500 colonies blanches, correspondant théoriquement à des microorganismes recombinants, ont été obtenus sur milieu X-gal. Ces colonies ont été repiquées individuellement, transférées sur membrane de nylon, puis analysées par hybridation avec la sonde sp 918 marquée au ³²p. Deux clones ont ainsi été repérés comme hybridant très fortement avec la sonde, et ont été isolés et retenus pour la poursuite du clonage.

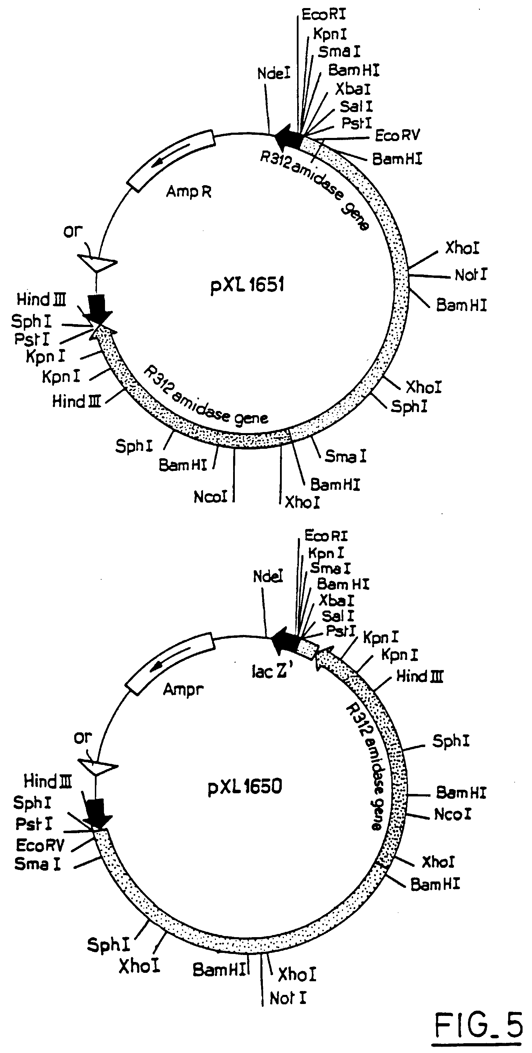

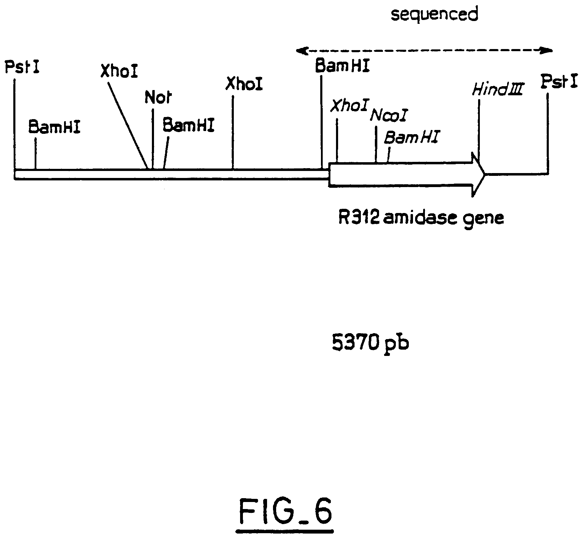

Les deux plasmides recombinants pXL1650 ET pXL1651 isolés à partir de ces deux clones ont été analysés par diverses méthodes : cartographie de restriction, séquençage partiel en utilisant les sondes comme amorce de séquençage et Southern Blot. Les résultats présentés à la figure 5 montrent que les deux plasmides contiennent le même insert PstI de 5,4 kpb environ, dans les deux orientations. La figure 6 présente une carte de restriction de ce fragment. Ces deux plasmides contiennent bien les séquences codant pour les peptides caractérisés, le fragment trypsique jouxtant l'extrêmité N-terminale (figure 9). De plus, ces résultats montrent que le gène codant pour l'amidase énantiosélective de Brevibacterium R312 est localisé sur un fragment BamHI-PstI de 2,4 kpb environ, et orienté dans le sens BamHI vers PstI. Etant donné la position de l'extrêmité 5′ de la séquence codante et sachant que l'enzyme est codée par, au maximum, 2 kpb (monomère de 57-63 KD suivant les estimations), il était donc certain que le gène entier était contenu dans le fragment BamHI-PstI que l'on a donc entrepris de séquencer.

### Exemple 3 - Séquence du fragment BamHI-PstI contenant le gène de l'amidase énantiosélective de Brevibacterium R312

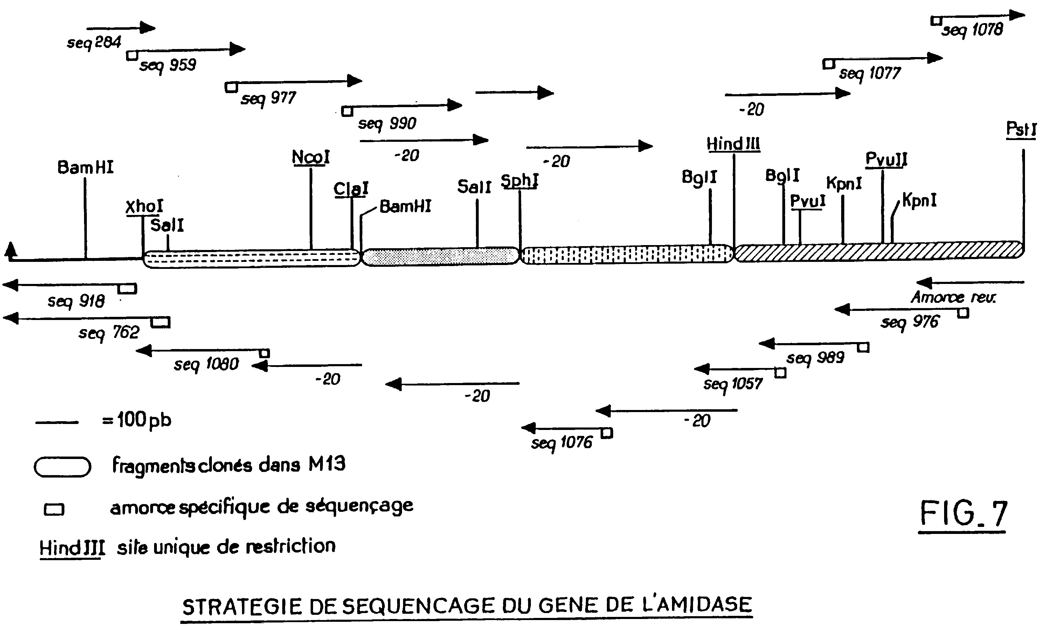

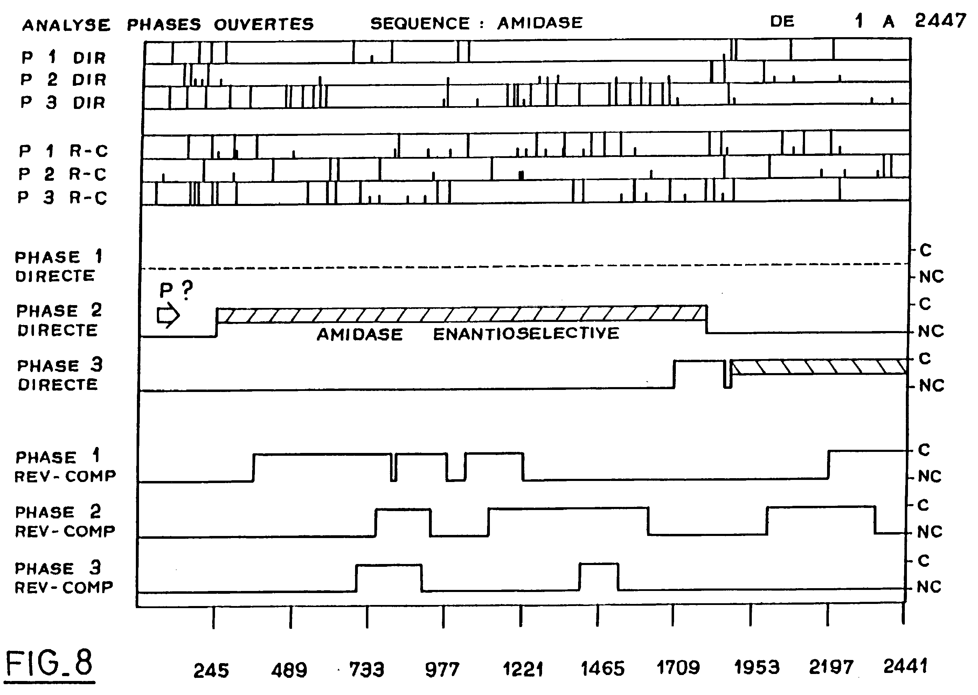

La stratégie de séquençage du fragment BamHI-PstI est indiquée sur la figure 7. Les diverses séquences ont toutes été obtenues par la méthode de terminaison de chaînes (Kit séquenase en présence de 7-deaza dGTP ; (S³⁵)dATP) soit sur des matrices simple brin de M13 recombinant portant des sous-fragments, soit directement sur le plasmide pXL1650. Plusieurs amorces spécifiques ont également été synthétisées dans ce but. le GC% moyen de la séquence obtenue est de 61,5%. Une analyse des phases ouvertes obtenues est présentée sur la figure 8. Cette figure montre que la phase ouverte correspondant au N-terminal de l'amidase code pour 521 acides aminés correspondant à un poids moléculaire de 54671. On constate sur cette phase ouverte que le GC% est respectivement de 65,8, 52,5 et 70% pour la première, deuxième et troisième position de codons, ce qui est une répartition caractéristique dans les séquences codantes de microorganismes riches en GC. La séquence complète du fragment BamHI-PstI est donnée à la figure 9.

### Exemple 4 - Expression du gène de l'amidase énantiosélective de Brevibacterium R312 dans E.coli

### 4.1. - Construction des plasmides

Plusieurs constructions ont été réalisées dans lesquelles le gène structural de l'amidase, contenant un site de fixation de ribosomes homologue ou dérivé du gène CII du phage lambda, est placé sous contrôle de son propre promoteur, du promoteur de l'opéron tryptophane ou du promoteur droit du phage lambda thermosensible. Le plasmide pXL1650 (figure 5) a été obtenu par insertion dans l'unique site PstI du plasmide pUC19, du fragment 5,4 kpb résultant de la digestion par PstI de l'ADN génomique total de Brevibacterium R312. Ce plasmide contient donc le promoteur de l'opéron lactose Plac, suivi du site de fixation des ribosomes et du gène structural de l'amidase énantiosélective de Brevibacterium R312, ainsi qu'un gène de résistance à l'ampicilline.

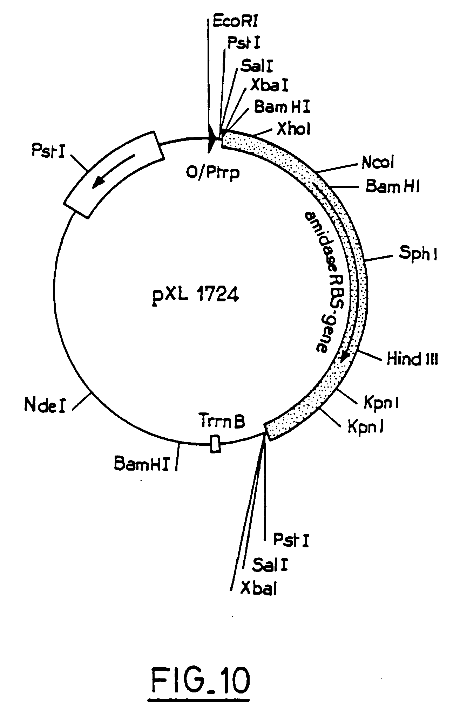

Le plasmide pXL1724 (figure 10) a été obtenu en insérant dans un vecteur contenant le promoteur de l'opéron tryptophane, le fragment BamHI-PstI de 2,26 kpb, excisé par traitement à l'enzyme BamHI du fragment de 5,4 kpb. Ce fragment contient le gène complet de l'amidase énantiosélective de Brevibacterium R312, précédé des 58 paires de bases en amont du codon ATG, portant le site de fixation des ribosomes.

Deux autres constructions ont été réalisées dans lesquelles le gène structural de l'amidase énantiosélective de Brevibacterium R312 est placé sous le contrôle de promoteurs hétérologues et de sites de fixation des ribosomes hétérologues.
Ces plasmides (pXL1751 et pXL1752) ont été obtenus de la manière suivante :

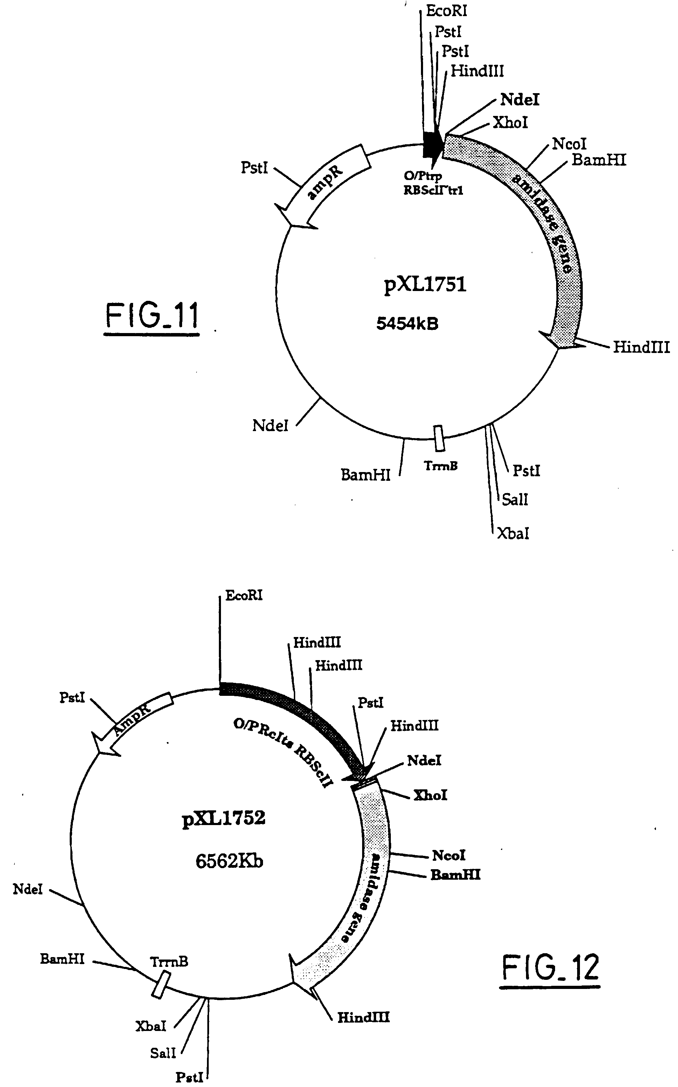

Le plasmide pXL1724 a été mutagénisé par PCR de manière à introduire, à la place du codon ATG situé en amont du gène structural de l'amidase, un site de coupure NdeI : CATATG. L'amplification a été réalisée en utilisant une amorce correspondant au site de coupure NdeI hybridant avec le codon d'initiation ATG et une amorce correspondant au site de coupure XhoI situé quelques paires de bases en aval du codon ATG. Le fragment amplifié a ensuite été excisé par coupure avec les deux enzymes NdeI et XhoI.
- Utilisation du promoteur Ptrp :
   Un fragment EcoRI-NdeI contenant le promoteur Ptrp et le site de fixation des ribosomes du gène CII de lambda, ne contenant pas la séquence de terminaison tR₁ et la région 5' du gène structural de l'amidase a été inséré dans le plasmide pXL1724 ouvert par EcoRI et XhoI, pour générer le plasmide pXL1751 (figure 11).
- Utilisation du promoteur PRCIts :
   La même stratégie a été employée en utilisant cette fois le fragment EcoRI-NdeI du plasmide pXL820 contenant le promoteur PRCIts et le site de fixation des ribosomes du gène CII de lambda ne contenant pas la séquence tRI. Le plasmide pXL1752 a ainsi été obtenu (figure 12).

### 4.2 - Expression de l'amidase de Brevibacterium R 312 dans coli B et E103S

Les plasmides pXL1751 et pxL1752 ont été utilisés pour transformer les souches de coli B et E103s respectivement selon la méthode du chlorure de calcium. La sélection des microorganismes recombinants se fait sur milieu ampicilline.

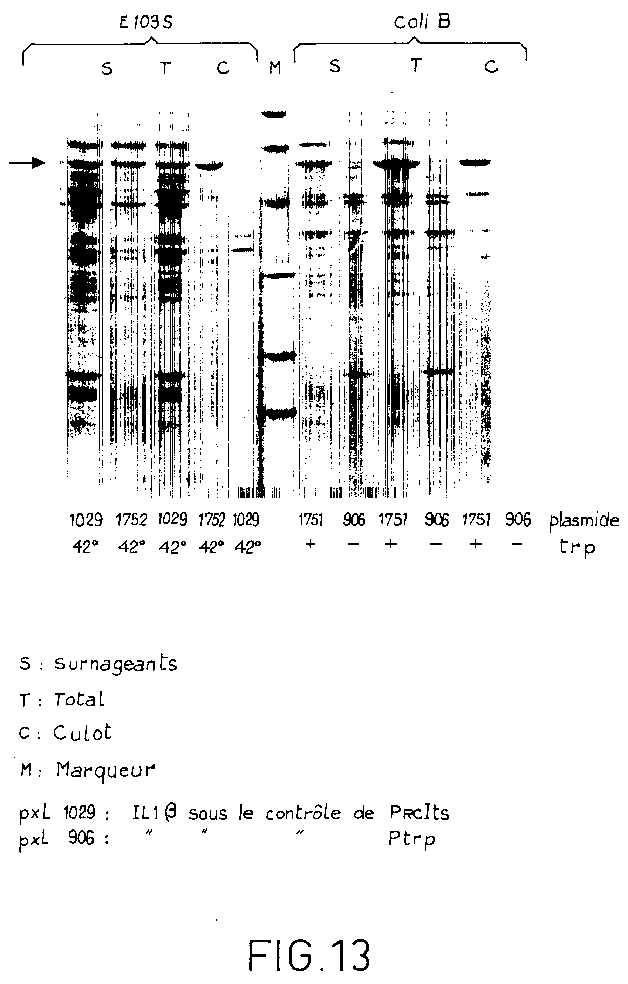

L'expression de l'amidase énantiosélective de Brevibacterium R 312 a été mesurée après sonication des cellules en gel SDS-PAGE, dans la fraction brute ou, après centrifugation, dans le culot et dans le surnageant. Les résultats sont présentés à la figure 13 et montrent un niveau élevé d'expression de l'amidase qui représente jusqu'à 20 % des protéines totales.

### EXEMPLE 5 : Purification de l'amidase énantiosélective d'un Rhodococcus.

### I. Dosage de l'activité enzymatique

La fraction contenant l'activité est incubée durant 30 min à 30°C dans 500 µl de tampon Tris/HCl 0.1MpH 7.5 contenant 5mM DTT et 18.1 mM phényl-2-propionamide. Après incubation, 2 ml de mélange acétonitrile/HCl 1 N (90/10) puis 2 ml de mélange H₃PO₄ 50 mM/CH₃CN (75/25) sont ajoutés au mélange d'incubation. Après centrifugation à 5000 g durant 10 min, une aliquote du surnageant est injectée en HPLC pour doser les produits de la réaction.
- Colonne : Nucléosil 5-C18 25 cm
- Eluant : H₃PO₄ 50 mM/CH₃CN (75/25)
- Injection : 10 µl
- Débit : 1 ml/min

L'unité d'activité est définie comme la quantité d'enzyme nécessaire pour hydrolyser 1 µ mole de phényl-2-propionamide par h.

### II. Protocole de purification

### 1. Préparation de l'extrait enzymatique

7 g de cellules sont suspendues dans 15 ml de tampon Tris/HCl 0.1 M pH 7.5-5 mM DTT et cassées par sonication pendant 15 min à 4°C. L'extrait enzymatique brut est récupéré par centrifugation 1 h à 50000 g.

### 2. Première chromatographie d'échange d'ions

A cet extrait brut (20 ml) sont ajoutés 20 ml de tampon A : Tris/HCl 25 mM pH 7.5-5 mM DTT. L'échantillon est injecté à un débit de 3 ml/min sur une colonne Mono Q HR 10/10 (Pharmacia) équilibrée dans le tampon A. Après lavage de la colonne, les protéines sont éluées avec un gradient linéaire de KCl (0 à 1M) développé en 1 h à 3 ml/min. Des fractions de 6 ml sont recueillies. L'amidase est éluée sur 18 ml avec 0.3 M de KCl environ.

### 3. Seconde chromatographie d'échange d'ions

Les fractions contenant l'activité sont rassemblées et concentrées à 2 ml à l'aide d'un module d'ultrafiltration Centriprep (Amicon). Après dilution avec 6 ml de tampon A, 4 ml de cet échantillon sont injectés à 1 ml/min sur une colonne Mono Q HR 5/5 équilibrée dans le tampon A. Les protéines sont éluées avec un gradient linéaire de KCl (0 à 0.5 M) dans le tampon A. Les fractions sont regroupées, l'échantillon est amené à 15 % de glycérol (vol/vol) et est finalement concentré à 1 ml comme ci-dessus.

### 4. Chromatographie hydrophobe

1 ml de tampon Tris/HCl 0.1 M pH 7.5-0.5 mM DTT-1.7 M (NH₄)₂SO₄ (tampon B) est ajouté à l'échantillon qui est ensuite injecté (en deux fois) sur une colonne de phényl-Superose HR 5/5 (Pharmacia) à un débit de 0.25 ml/min. Les protéines sont éluées à 0.5 ml/min avec un gradient linéaire croissant de (NH₄)₂SO₄ ( 0 M à 1,7 M) sur 25 ml. Des fractions de 0.5 ml sont collectées. La fraction active est amenée à 15% de glycérol puis diluée avec 1 ml de tampon A.

### 5. Chromatographie sur hydroxyapatite

L'échantillon est injecté à 0.5 ml/min sur une colonne Bio-Gel HPHT (Bio-Rad) équilibrée dans le tampon Tris/HCl 85 mM pH 7.5-0.5 mM DTT-10 µM CaCl₂-15 % glycérol (tampon C). L'amidase est éluée à un débit de 0.5 ml/min avec un gradient linéaire de 0 à 100 % de tampon phosphate de potassium 0.35 M pH 7.5-0.5 mM DTT-10 µM CaCl₂-15% glycérol dans le tampon C en 20 min.

Ces différentes étapes permettent d'obtenir une enzyme purifiée à homogénéité avec une activité spécifique de 988 U/mg de protéine.

Comme celle de Brévibactérium R 312, l'enzyme ainsi obtenue se comporte comme un dimère de sous-unités identiques d'un poids moléculaire apparent de 53 KD +/- 2 KD.

### EXEMPLE 6 Clonage du gène de l'amidase obtenue à l'exemple 5

Après une nouvelle étape de purification sur TSK-G3000 SW, l'enzyme a fait l'objet d'un travail de séquençage. L'extrémité N-terminale étant inaccessible à la chimie d'Edman, une hydrolyse totale à la trypsine a été effectuée et trois fractions HPLC de l'hydrolysat : 123, 124, et 162 ont donné des peptides permettant d'obtenir une séquence non ambigüe. A partir de ces données, trois sondes nucléotidiques 32-mer, correspondant aux mélanges de 8 à 16 oligonucléotides et contenant chacune 7 inosines dans les positions au moins 3 fois dégénérés, ont été synthétisées :
SONDE A (peptide 123) 
A T V D V P V P D Y A

5'                                                    3'

GCIACIGTIGATGTICCIGTICCIGATTATGC

           C              C  C
SONDE B (peptide 124) 
E A G E L V P A T D Y
5' 3'
GAAGCIGGIGAACTIGTICCIGCIACIGATTA
G GT C
SONDE C (peptide 162)

5'                                                    3'
CAAGATATIGATGTICTTIATIGCICCIACIGT
G  C     C  T

L'efficacité de ces sondes, marquées en 5′ au ³²p, a ensuite été testée par transfert de Southern sur de l'ADN génomique de Rhodococcus préalablement digéré par l'une des enzymes de restriction suivantes : SstI, SphI, SmaI, PstI, KnpI, EcoRI SalI et BamHI. Les conditions expérimentales étaient les suivantes : tampon d'hybridation : 5x SSC, 5x Denhardt, 0,1 % SDS, 50 mM Na₃PO₄ pH 6,5, 250 µg/ml ssDNA ; températures d'hybridation : 50 ou 55°C (2 expériences) ; conditions de lavages : 1 h, 6x SSC, Température ambiante et 5 min en 2x SSC, 0,1 % SDS à 50°C.

Dans ces conditions, la sonde A nous a permis d'obtenir des signaux importants et sans ambiguité ; en particulier, dans le cas des digestions par BamHI, KpnI, SphI, SstI, SmaI, SalI et PstI, une seule bande génomique a été trouvée hybridant fortement à A correspondant au fragment génomique PstI de ≈ 3,2 kb.

Les fragments de 3 à 4 kb d'une digestion PstI de l'ADN génomique ont été purifiés par électrophorèse préparative sur agarose et électroélution, puis ligaturés au plasmide pUC19, lui-même digéré par PsTI. Après transformation dans la souche DH5α, 600 clones blancs sur LB Amp X-gal ont été repiqués individuellement et sondés par hybridation sur colonie avec la sonde A, dans des conditions de stringence semblables à celles utilisées en Southern. Neuf clones ayant donné des signaux d'hybridation particulièrement forts ont ensuite fait l'objet d'une analyse par restriction de l'ADN plasmidique. Parmi six de ces clones ayant manifestement inséré le même fragment ≈ 3,2 kb dans les deux orientations, deux clones représentant chacune les deux orientations (pXL1835 et pXL1836) ont été analysés plus en détail (carte détaillée, analyse par Southern), confirmant ainsi que le fragment recherché avait bien été obtenu.

### EXEMPLE 7 Séquence du fragment PstI de 3,2 kb.

La séquence nucléotidique complète du fragment PstI de 3,2 kb a été déterminée sur les deux brins (voir figure 14). Le pourcentage en G + C de ce fragment s'élève à 62,4 %, soit du même ordre que le pourcentage observé cher R312 (≈ 62 %). L'analyse de la séquence obtenue a permis de caractériser une phase ouverte de 1386 nucléotides (position 210 à 1595) codant pour un polypeptide de 462 résidus (mr 48554) contenant les 3 séquences peptidiques obtenues par séquençage de fragments trypsiques.

Cette phase ouverte représente le gène structural de l'amidase énantiosélective recherchée.

### EXEMPLE 8 Homologies entre différentes amidases : Identification d'une séquence caractéristique de l'activité amidase.

Nous avons tout d'abord comparé les séquences peptidiques des amidases énantiosélectives de R312 et de celle présentée sur la figure 14.

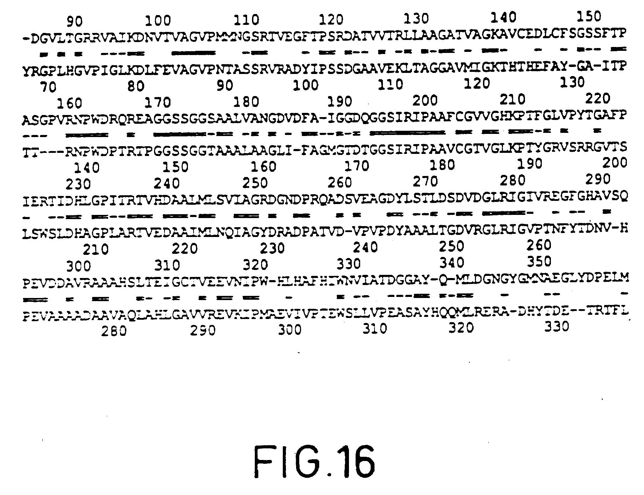

La figure 16 montre que les deux protéines sont particulièrement homologues (50 % d'identité stricte) dans le deuxième tiers de la séquence, entre les résidus 150 et 300 de R312 et 67 % dans la zone 158-215.

Nous avons par ailleurs effectué une recherche dans la banque GENPRO de séquences homologues. Cette recherche fait apparaître des homologies importantes dans la région 150-200, avec les séquences de l'acétamidase de Aspergillus nidulans, de l'indolacétamide hydrolase de Pseudomonas syringae et de Bradyrhizobium japonicum, de la protéine tms2 de Agrobactérium tumefaciens, et des 6-amino- hexanoate cyclic dimerhydrobases (ACDH) de Flavobactérium sp K172 et Pseudomonas sp NK87 (voir en particulier la figure 17).

L'homologie du peptide 137-193 de l'amidase décrite dans la présente demande avec les sites respectifs de ces autres enzymes (en % d'identité stricte des acides aminés) est donnée dans le tableau suivant :

| AMIDASE | % HOMOLOGIE |
|---|---|
| R 312 | 65,5 |
| IAH A. tumefaciens | 64,3 |
| IAH P. syringae | 61,8 |
| ACDH (F. ou P.) | 61,4 |
| IAH B. japonicum | 54,4 |
| Acetamidase (A. nidulans) | 47,4 |

Cette région, fortement conservée, est vraisemblablement responsable de l'activité de ces enzymes.

### EXEMPLE 9 Expression de l'amidase énantiosélective dans E. Coli

Afin de confirmer l'identification de la phase codant pour une amidase énantiosélective, un site NdeI (CATATG) a été créé par PCR au niveau de l'ATG présomptif en 210 (figure 14) et le fragment compris entre ce site et le site SalI (position 1683), contenant uniquement la partie codant pour l'amidase, a été placé sous le controle de signaux efficaces de démarrage de la transcription (promoteurs Ptrp ou PR) et de la traduction (RBS cII) chez E. Coli. Les vecteurs ainsi obtenus : pXL1893 (Ptrp) et pXL1894 (PR-cI^{ts}) sont semblables aux vecteurs pXL1752 et pXL1751 décrits précédemment, exprimant l'amidase de R312. La structure générale de ces vecteurs d'expression est rappelée sur la figure 18. L'expression à partir des plasmides pXL1893 et pXL1894 a été étudiée respectivement dans les souches E. Coli B et K12 E103S. Les résultats obtenus dans le cas de pXL1894 sont représentés sur la figure 19. Une protéine co-migrant avec l'amidase purifiée est spécifiquement produite à 42°C, en présence du plasmide pXL1894.

### EXEMPLE 10 Expression de l'amidase chez les corynébactéries.

### A) Construction des vecterus d'expression :

Ces vecteurs sont élaborés à partir de vecteurs réplicatifs chez les corynébactéries, et comprennent :
- un réplicon d'E. Coli
- un réplicon de corynébactérie
- un marqueur de sélection, et
- une séquence Amd.

. Vecteur pSV73 (figure 20) : Ce plasmide dérive du vecteur pSR1 de C. glutamicum (Yoshihama et coll. J. Bacteriol. 162, 591 (1985)) par insertion d'un fragment du plasmide pUC8 contenant un réplicon d'E. Coli et du gène de résistance à la kanamycine provenant du transposon Tn903.

Ce plasmide a été utilisé pour construire différents vecteurs d'expression des séquences Amd données aux figures 14 et 15.
Notamment :
. Vecteurs pYG811A et B (figure 21) : Ces vecteurs dérivent du vecteur pSV73 par clonage au site SalI, dans les deux orientations, de la séquence Amd donnée à la figure 14.
. Vecteurs pYG817A et B (figure 21) : Ces vecteurs dérivent du vecteur pSV73 par clonage au site BglII, dans les deux orientations, de la séquence Amd donnée à la figure 14.
. Vecteur pYG822 (figure 20) : Ce vecteur dérive de pSV73 par clonage aux sites SalI-BglII d'une cassette d'expression contenant la séquence Amd de la figure 14 et le promoteur Ptrp du bactériophage lambda.

D'autres plasmides cryptiques de corynébactérie peuvent également être utilisés pour construire des vecteurs d'expression de séquences Amd dans les corynébactéries. Notamment le Plasmide pX18 isolé chez B. lactofermentum (Yoshihama et coll. précitée) a permis la construction du vecteur navette pYG820A dont la carte de restriction est donnée sur la figure 20.

### B) Transformation des corynébactéries

Toutes les techniques connues de l'homme de l'art peuvent être utilisées, et notamment, la technique de protoplastisation-régénération décrite par Yoshihama et coll. précitée. Cependant, la demanderesse a montré que la technique d'électroporation est très avantageuse, puisqu'elle permet d'augmenter jusqu'à 1000 fois la fréquence de transformation.

L'analyse en polyacrylamide SDS des surnageants de cultures soniquées et centrifugées indique la présence de transformants.

### EXEMPLE 11 Catalyse enzymatique

Cet exemple illustre l'utilisation selon l'invention des polypeptides ou des microorganismes recombinés préparés aux exemples précédents, pour synthétiser de l'adipate d'ammonium par hydrolyse de l'adipamide ou de l'adipamate d'ammonium.

### A) Milieux de culture des souches utilisées

### 1- pour les souches naturelles

**Milieu 1 :** pour Brévibactérium R312
   . glucose 10g/l
   . (NH₄)₂SO₄ 5g/l
   . KH₂PO₄ 1,01g/l
   . Na₂HPO₄, 12H₂O 1,64g/l
   . K₂HPO₄ 0,82g/l
   . CaCl₂, 2H₂O 0,012g/l
   . Zn Cl₂ 0,0012g/l
   . FeSO₄, 7H₂O 0,0012g/l
   . MnSO₄, H₂O 0,0012g/l
   . MgSO₄, 7H₂O 0,5g/l
   . chlorhydrate de thiamine 0,002g/l
   . eau distillée
**Milieu 2 :** pour Rhodococcus
   . Glycérol 5g/l
   . Yeast Extract (Difco) 1 g/l
   . Beef Extract (Difco) 1g/l
   . K₂ HPO₄ 2g/l
   . MgSO₄, 7H₂O 0,5g/l
   . Fe SO₄, 7H₂O 20mg/l
   . MnSO₄, H₂O 20mg/l
   . NaCl 10mg/l
   . Solution minérale * 10mg/l
   . NaOH q.s.p. pH 7,2
   . isobutyronitrile 5g/l
   * Solution minérale :
   . CaCl₂, 2H₂O 200mg/l
   . Na₂ MoO₄, 2H₂O 15mg/l
   . Zn SO₄, 7H₂O 4mg/l
   . Cu SO₄, 5H₂O 0,4mg/l
   . CoCl₂, 4H₂O 0,4mg/l
   . H₂ BO₃ 20mg/l
   . KI 10mg/l
   . HCl 10 % 10mg/l

### 2 - Pour les souches recombinantes

**Milieu 3 :**
   . NaCl 5g/l
   . Bacto-tryptone 10g/l
   . Extrait de levure 5g/l
   . Isobutyronitrile 5g/l
   . Kanamycine 1g/l
**Milieu 4 :**
   . NaCl 5g/l
   . Bacto-tryptone 10g/l
   . Extrait de levure 5g/l
   . Isobutyronitrile 2,5g/l
   . Isobutyramide 2,5g/l
   . Kanamycine 20mg/l
**Milieu 5 :**
   . Na₂HPO₄ 7g/l
   . KH₂PO₄ 3g/l
   . NaCl 0,5g/l
   . NH₄Cl 1g/l
   . chlorhydrate de thiamine 0,01g/l
   . glucose 4g/l
   . MgSO₄ 1mM
   . CaCl₂ 0,1mM
   . Ampicilline 100 µg/ml
**Milieu 6 :**
   . milieu 5
   . tryptophane 40mg/l

### B) Préparation des culots cellulaires

Les cultures des différentes souches sont réalisées en erlens de 2 litres, remplis avec 600 ml de milieu, à 30°C sur table d'agitation (150 tr/mn). En fin de culture, les cellules sont récoltées, lavées avec du serum physiologique, réparties en tube Eppendorf et conservées à -18°C jusqu'à utilisation. Les milieux et caractéristiques des cultures des différentes souches testées sont données au tableau I ci-après.

### C) Mesure de l'activité amidase

Le protocole est le suivant :

Dans un flacon muni d'un agitateur, sont introduits l'adipamide ou l'adipamate d'ammonium, la suspension cellulaire et le tampon phosphate de potassium 50 mM pH 7,0.

Le flacon bouché est placé dans un cristallisoir thermostaté à 25°C sous agitation pendant toute la durée de la réaction.

Le mélange réactionnel est ensuite dilué avec de l'acide chlorhydrique 0,1 N.

Les bactéries et débris cellulaires sont éliminés par centrifugation suivie d'une filtration (0,45 µm).

La composition en acide adipique, adipamide et/ou acide adipamique est déterminée en CLHP.

Les résultats obtenus ainsi que les charges utilisées sont donnés dans le tableau II ci-après.
Dans ce tableau :
E = cellule entière
S = cellule soniquée
IBN = isobutyronitrile
IBAm = isobutyramide
NMA = N - méthylacétamide
Trp = Tryptophane
- = néant

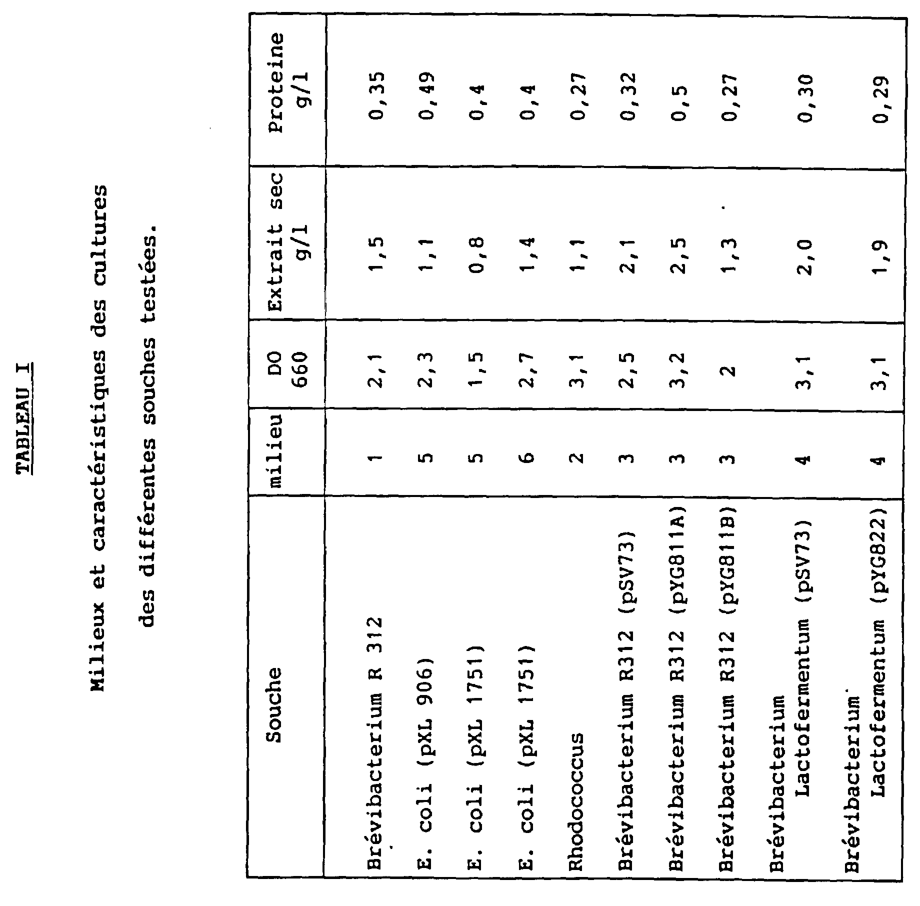

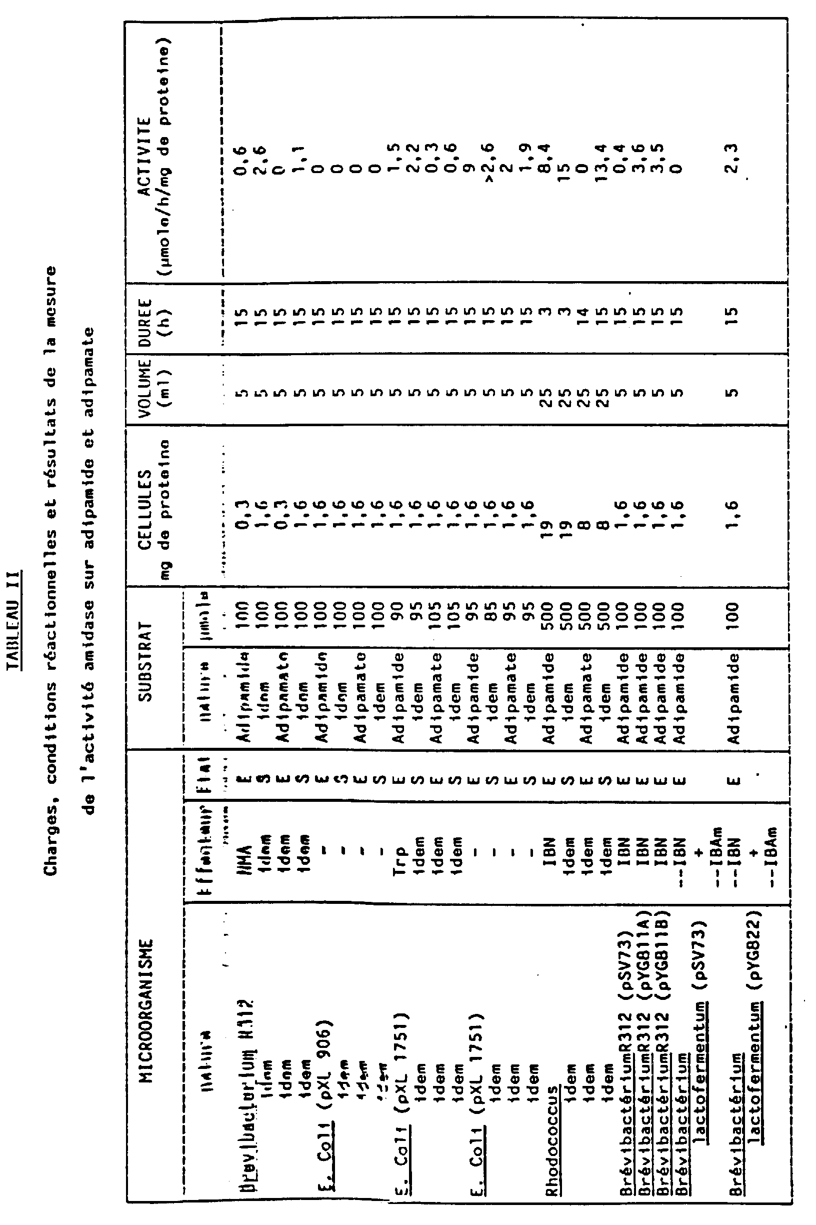

## Revendications

1. Procédé de synthèse d'adipate d'ammonium par hydrolyse de l'adipamide et/ou de l'adipamate d'ammonium au moyen d'un polypeptide ayant une activité amidase, ou d'un microorganisme recombiné générant ledit polypeptide,
caractérisé par le fait que l'on utilise :
(i) un polypeptide codé par une séquence d'**ADN** choisie parmi :
- la séquence codant pour l'amidase de *Brévibactérium* **R 312** représentée à la **fig. 9**,
- la séquence représentée à la **fig. 14**,
- un analogue de ces séquences résultant de -la dégénérescence du code génétique,
- un **ADN** hybridant avec l'une de ces séquences ou avec un fragment de celles-ci et codant pour un polypeptide ayant une activité amidase,
(ii) ou un microorganisme recombiné générant ledit polypeptide.

2. Procédé selon la revendication 1, caractérisé en ce que le microorganisme hôte est une entérobactérie.

3. Procédé selon la revendication 2, caractérisé en ce que l'entérobactérie est *E. coli.*

4. Procédé selon la revendication 1, caractérisé en ce que le microorganisme hôte est une bactérie corynéforme.

5. Procédé selon la revendication 4, caractérisé en ce que cette bactérie appartient aux genres *Corynebactérium, Brévibactérium* ou *Rhodococcus.*

6. Procédé selon l'une quelconque des revendications précédentes, caractérisé en ce que le polypeptide ou le microorganisme recombiné sont immobilisés sur et/ou dans un support solide.

## Patentansprüche

1. Verfahren zur Synthese von Ammoniumadipat durch Hydrolyse von Adipamid und/oder Ammoniumadipamat mit einem Polypeptid, das eine Amidase-Aktivität aufweist, oder einem rekombinierten Mikroorganismus, der dieses Polypeptid erzeugt,
dadurch gekennzeichnet, daß
verwendet wird:
(i) ein Polypeptid, das durch eine DNS-Sequenz codiert wird, die ausgewählt ist unter:
- der in Fig. 9 dargestellten Sequenz, welche die Amidase von *Brevibacterium* R 312 codiert,
- der in Fig. 14 dargestellten Sequenz,
- Analoga dieser Sequenzen, die aus der Degeneration des genetischen Codes resultieren,
- DNS, die mit einer dieser Sequenzen oder mit einem Fragment dieser Sequenzen hybridisieren und ein Polypeptid codieren, das eine Amidase-Aktivität aufweist,
oder
(ii) ein rekombinierter Mikroorganismus, der dieses Polypeptid erzeugt.

2. Verfahren nach Anspruch 1, dadurch gekennzeichnet, daß der Wirts-Mikroorganismus ein Enterobakterium ist.

3. Verfahren nach Anspruch 2, dadurch gekennzeichnet, daß das Enterobakterium *E.coli* ist.

4. Verfahren nach Anspruch 1, dadurch gekennzeichnet, daß der Wirts-Mikroorganismus ein coryneformes Bakterium ist.

5. Verfahren nach Anspruch 4, dadurch gekennzeichnet, daß das Bakterium zur Gattung *Corynebacterium, Brevibacterium* oder *Rhodococcus* gehört.

6. Verfahren nach einem der vorhergehenden Ansprüche, dadurch gekennzeichnet, daß das Polypeptid oder der rekombinierte Mikroorganismus auf und/oder in einem festen Träger immobilisiert sind.

## Claims

1. A method of synthesizing ammonium adipate by hydrolyzing adipamide and/or ammonium adipamate by means of a polypeptide having amidase activity or by means of a recombined microorganism generating said polypeptide,
characterized by the fact that the following are used:
(i) a polypeptide coded by a DNA sequence selected from:
the sequence coding adimase of *Brevibacterium* R 312, shown in Figure 9;
the sequence shown in Figure 14;
an analog of these sequences resulting from degeneracy of the genetic code; and
a DNA that hybridizes with one of these sequences or with a fragment thereof and codes for a polypeptide having amidase activity;
(ii) or a recombined microorganism generating said polypeptide.

2. A method according to claim 1, characterized in that the host microorganism is an enterobacterium.

3. A method according to claim 2, characterized in that the enterobacterium is *E. coli.*

4. A method according to claim 1, characterized in that the hose microorganism is a coryneform bacterium.

5. A method according to claim 4, characterized in that said bacterium belongs to the genera *Corynebacterium, Brevibacterium,* or *Rhodococcus.*

6. A method according to any preceding claim, characterized in that the polypetide or the recombined microorganism are immobilized on and/or in a solid support.
